# EUROPEAN PATENT SPECIFICATION

(11) **EP 3 092 963 B1**
(45) Date of publication and mention of the grant of the patent: **12.07.2017**
(21) Application number: 15167435.5
(22) Date of filing: 12.05.2015
(51) Int. Cl.: A61B 17/70

(54) **COUPLING DEVICE FOR COUPLING A ROD TO A BONE ANCHORING ELEMENT AND BONE ANCHORING DEVICE WITH SUCH A COUPLING DEVICE**
KOPPLUNGSVORRICHTUNG ZUM KOPPELN EINER STANGE AN EIN KNOCHENVERANKERUNGSELEMENT UND KNOCHENVERANKERUNGSVORRICHTUNG MIT SOLCH EINER KOPPLUNGSVORRICHTUNG
DISPOSITIF DE COUPLAGE D'UNE TIGE À UN ÉLÉMENT D'ANCRAGE OSSEUX ET DISPOSITIF D'ANCRAGE OSSEUX AVEC UN TEL DISPOSITIF

(43) Date of publication of application: 16.11.2016
(73) Proprietor: Biedermann Technologies GmbH & Co. KG, 78166 Donaueschingen (DE)
(72) Inventor: Biedermann, Lutz, 78048 VS-Villingen (DE); Biedermann, Timo, 78647 Trossingen (DE); Matthis, Wilfried, 79367 Weisweil (DE)
(74) Representative: Prüfer & Partner mbB Patentanwälte · Rechtsanwälte

(56) References cited:
- EP-A1- 2 851 021
- US-A- 5 882 350
- US-A1- 2007 167 949
- US-A1- 2012 046 701
- US-A1- 2014 257 411

## Description

The invention relates to a coupling device for coupling a rod to a bone anchoring element. The coupling device includes a receiving part for receiving a rod for coupling the rod to a bone anchoring element wherein the receiving part is configured to accommodate a head of a bone anchoring element such that the head can pivot with respect to the receiving part. The coupling device further includes a pressure element with a flexible portion to clamp an inserted head and a clamping element that is configured to exert a compression force onto the pressure element to increase a friction force between the pressure element and the head.

US 8,926,671 describes a receiving part for receiving a rod for coupling the rod to a bone anchoring element wherein the receiving part comprises a receiving part body for accommodating a head of a bone anchoring element and a pressure element with a flexible portion to clamp an inserted head. The pressure element is movable along a longitudinal axis of the receiving part body from an insertion position to insert the head and to a pre-locking position wherein the head is clamped in the receiving part by a pre-stress exerted by the pressure element, to a locking position to lock the head in the receiving part. The pre-stress exerted by the pressure element allows to maintain a desired angular position of the bone anchoring element relative to the receiving part by friction before the head of the bone anchoring element is finally locked.

US 6,248,105 B1 describes a device for connecting a longitudinal rod with a bone anchoring element, such as a pedicle screw, thereby forming a system for fixation of the spine. The device includes a connecting member accepting a longitudinal rod. Within the connecting member a radially compressible spring chuck is arranged that has a cavity for receiving the head of the bone anchoring element. An insert slides into a bore hole of the connecting member and has a recess with a shape complementarily conical to the cone at the spring chuck. The insert radially compresses the spring chuck via the conical joining and therewith fastens the head of the pedicle screw.

US 8,951,294 B2 describes a spinal implant with an anchoring part and a mounting part with an internal axial housing to receive transversely a connecting rod. The spinal implant includes retaining elements that are situated near the bottom of the axial housing of the mounting part and that are adapted to fasten a locking member in a stationary so-called locking position in which a ball joint connection formed by first and second connecting elements is locked to fasten the anchoring part and the mounting part in rotation relative to each other about at least two orthogonal axes.

US 2013/0096622 A1 describes a polyaxial bone anchoring device wherein a pressure element exerts pressure onto the head that maintains the head in an adjustable angular position relative to a receiving part by friction using set screws. By controlling the thread turning and advancement of the set screws a desired friction force can be achieved.

US 2007/0167949 A1 discloses a screw system including a seat, a coupler to snap-fit over a head of a screw, and a retaining ring to secure the coupler inside the seat. The retaining ring is seated upon a lip of the coupler, wherein projections of the retaining ring are received by keyways of the coupler and press-fitted into keyways of the seat in order to orient and engage the retaining ring with respect to the coupler and the seat.

US 5,882,350 discloses a polyaxial orthopedic device including a screw, a two-piece interlocking coupling element which mounts about a screw head, and a rod receiving cylindrical body having a tapered lower portion into which both the screw and the coupling element are securely nested. The two pieces of the coupling element are a cap and a socket inserted into the cap. By locking a rod in a channel of the body, the cap is forced downwardly onto the socket, thereby driving the socket into the tapered lower portion of the body and causing it to compression-lock the screw head.

It is the object of the invention to provide an improved coupling device and an improved bone anchoring device that facilitates the handling during surgery.

The object is solved by a coupling device according to claim 1 and by a bone anchoring device according to claim 15. Further developments are given in the dependent claims.

The coupling device allows to adjust the friction force that is exerted onto the head of the bone anchoring element in an easy manner during surgery. The coupling device may be particularly useful in a polyaxial bone anchoring device of the bottom loading type, wherein the head of the bone anchoring element is inserted from a bottom end into the receiving part. More particularly, the coupling device allows to place the receiving part in-situ onto a head of a bone anchoring element wherein the shank has already been inserted into the bone. Hence, an in-situ type bottom loading bone anchoring element is provided wherein the friction force acting onto the head can be increased by actuating the clamping element. With the frictional clamping of the head before final locking the procedure of aligning a plurality of receiving parts for inserting a rod is considerably simplified.

In one embodiment, the clamping element cooperates with a portion of the receiving part according to an advancement structure of a bayonet locking type, wherein an engagement member such as a pin, engages a helical groove that allows to advance the clamping element in an axial direction while rotating it. The advancement is stepless, therefore a stepless adjustment of the clamping force is permitted The cooperation of the engagement member with the helical groove may be self-locking such that each position is secured against inadvertent movement.

In another embodiment, an advancement structure that permits an axial advancement of the clamping element is formed by a threaded connection between the clamping element and the receiving part. This also allows to advance the clamping element relative to the receiving part in a stepless manner and to adjust the friction force onto the pressure element in a stepless manner. A pitch of the threaded connection may be the same as a pitch of a threaded connection between the receiving part and a locking element so that the clamping element can be screwed into the receiving part in the same manner as the locking element.

In a further embodiment, the clamping element is an open ring similar to a snap ring that can exert a compression force onto the pressure element when it is placed around the pressure element. The clamping element comprises at its side facing towards the pressure element protrusions that are in a first condition resting in recesses of the pressure element and in a second condition moved out of the thereby and enhancing the clamping force onto the pressure element. Hence, the clamping force can be increased with only a minimal movement of the clamping element.

The pressure element may have at least one longitudinal slot to provide a flexible portion for clamping the head. In one embodiment, the pressure element may have an additional horizontal slot to reduce the insertion force of the head into the pressure element.

In a still further embodiment, the pressure element and the clamping element and preferably also the receiving part are manufactured as a monolithic piece, wherein the connection between the pressure element and the clamping element and preferably also between the clamping element and the receiving part has at least one predetermined breaking point. By exerting a force, for example by rotating the clamping element, the connection breaks at the predetermined breaking point. Thereafter, the pieces are separate parts. An additive manufacturing method can be used to manufacture the monolithic pieces, such as laser sintering or electron beam melting. Thereby, sophisticated structures may be produced.

Further features and advantages will become apparent from the description of embodiments by means of the accompanying drawings. In the drawings:
- Fig. 1: shows a perspective exploded view of a first embodiment of the bone anchoring device with a first embodiment of the coupling device.
- Fig. 2: shows a cross-sectional view of the bone anchoring device of Fig. 1 in an assembled state, the cross-section taken in a plane perpendicular to the rod axis.
- Fig. 3: shows a perspective view from the top of the receiving part of the coupling device according to the first embodiment shown in Figs. 1 and 2.
- Fig. 4: shows a perspective view from the bottom of the receiving part of Figs. 1 to 3.
- Fig. 5: shows a top view of the receiving part of Figs. 3 and 4.
- Fig. 6: shows a cross-sectional view of the receiving part of Figs. 3 and 4 along line A-A in Fig. 5.
- Fig. 7: shows a perspective view from the top of the pressure element of the coupling device according to the first embodiment shown in Figs. 1 and 2.
- Fig. 8: shows a perspective view from the bottom of the pressure element of Fig. 7.
- Fig. 9: shows a top view of the pressure element of Figs. 7 and 8.
- Fig. 10: shows a cross-sectional view of the pressure element of Figs. 7 to 9 along line B-B in Fig. 9.
- Fig. 11: shows a perspective view from the top of a clamping element of the coupling device according to the first embodiment of Figs. 1 and 2.
- Fig. 12: shows another perspective view of the clamping element of Fig. 11.
- Fig. 13: shows a top view of the clamping element shown in Figs. 11 and 12.
- Fig. 14: shows a cross-sectional view of the clamping element of Figs. 11 to 13 along line CC-CC in Fig. 13.
- Figs. 15a to 15c: show cross sectional views of steps of placing the coupling device according to the first embodiment shown in Figs. 1 to 14 onto a bone anchoring element.
- Figs. 15d to 15f: show cross-sectional views of steps of adjusting a clamping force onto an inserted head via the clamping element.
- Fig. 16: shows a perspective exploded view of a second embodiment of the bone anchoring device with a second embodiment of the coupling device.
- Fig. 17: shows a cross-sectional view of the bone anchoring device of Fig. 16, the cross-section taken in a plane perpendicular to the rod axis.
- Fig. 18: shows a partial cross-sectional view of the bone anchoring device shown in Figs. 16 and 17 without an inserted rod.
- Fig. 19: shows a perspective view from the top of the receiving part of the coupling device according to the second embodiment shown in Figs. 16 to 18.
- Fig. 20: shows a cross-sectional view of the receiving part of Fig. 19, the cross-section taken in a plane perpendicular to the rod axis.
- Fig. 21: shows a perspective view from the top of a clamping element of the coupling device according to the second embodiment as shown in Figs. 16 to 18.
- Fig. 22: shows a perspective view from the bottom of clamping element of Fig. 21.
- Fig. 23: shows a side view of the clamping element of Figs. 21 and 22.
- Fig. 24: shows a perspective exploded view of a third embodiment of the bone anchoring device with a third embodiment of the coupling device.
- Fig. 25: shows a cross-sectional view of the bone anchoring device of Fig. 24, the cross-section taken in a plane perpendicular to the rod axis.
- Fig. 26: shows a partial cross-sectional view of the bone anchoring device of Fig. 24 and 25.
- Fig. 27: shows a perspective view from the top of a pressure element of the coupling device according to the second embodiment shown in Figs. 24 to 26.
- Fig. 28: shows a perspective view from the bottom of the pressure element of Fig. 27.
- Fig. 29: shows a top view of the pressure element of Figs. 27 and 28.
- Fig. 30: shows a cross-sectional view of the pressure element of Figs. 27 to 29 along line D-D in Fig. 29.
- Fig. 31: shows a perspective exploded view of a fourth embodiment of a bone anchoring device with a fourth embodiment of a coupling device.
- Fig. 32: shows a cross-sectional view of the bone anchoring device of Fig. 31, the cross-section taken in a plane perpendicular to the rod axis.
- Fig. 33: shows a partial cross-sectional view of the bone anchoring device of Figs. 31 and 32 without an inserted rod.
- Fig. 34: shows a perspective view from the top of a receiving part of the coupling device of Figs. 31 and 32.
- Fig. 35: shows a perspective view from the bottom of the receiving part of Fig. 34.
- Fig. 36: shows a top view of the receiving part of Figs. 34 and 35.
- Fig. 37: shows a cross-sectional view of the receiving part of Figs. 34 to 36 along line E-E in Fig. 36.
- Fig. 38: shows a perspective view from the top of the pressure element of the fourth embodiment of Figs. 31 and 32.
- Fig. 39: shows a perspective view from the bottom of the pressure element of Fig. 38.
- Fig. 40: shows a top view of the pressure element of Figs. 38 and 39.
- Fig. 41: shows a cross-sectional view of the pressure element of Figs. 38 to 40 along line F-F in Fig. 40.
- Fig. 42: shows a perspective view from the top of a clamping element of the fourth embodiment as depicted in Figs. 31 and 32.
- Fig. 43: shows a perspective view from the bottom of the clamping element of Fig. 42.
- Fig. 44: shows a top view of the clamping element of Figs. 42 and 43.
- Fig. 45: shows a side view of the clamping element of Fig. 44.
- Figs. 46a to 46d: show steps of mounting the pressure element and the clamping element to the receiving part according to the fourth embodiment as depicted in Figs. 31 to 45.
- Figs. 46e to 46h: show steps of mounting the coupling device according to the fourth embodiment onto a bone anchoring element and adjusting the friction force with the clamping element.
- Fig. 47a: shows a perspective view from the top of a fifth embodiment of the coupling device.
- Fig. 47b: shows an enlarged view of a detail of Fig. 47a.
- Fig. 48: shows a cross-sectional view of the coupling device according to the fifth embodiment.
- Fig. 49: shows a top view of the coupling device of Figs. 47a to 48.
- Fig. 50: shows a cross-sectional view of the coupling device of Figs. 47 to 49 along line G-G in Fig. 49.

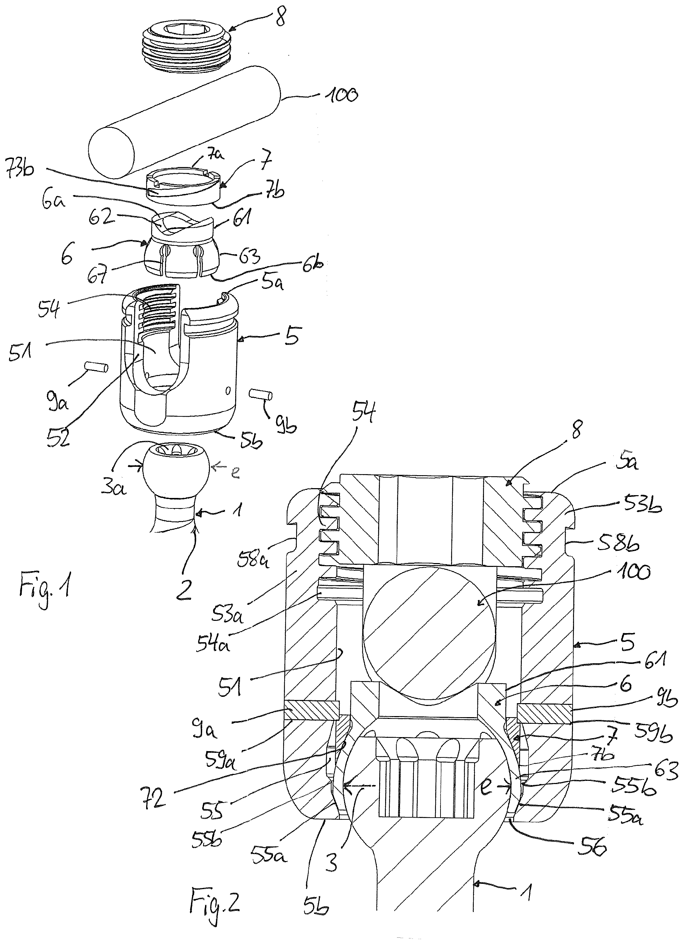

As shown in Figs. 1 and 2, a bone anchoring device according to a first embodiment comprises a bone anchoring element 1. The bone anchoring element 1 may be a bone screw having a threaded shaft 2 and a head 3 that may be a spherical segment-shaped head. The head 3 has a recess 3a for engagement with a screwing-in tool. The bone anchoring device further comprises a coupling device for receiving a rod 100 to connect the rod 100 to the bone anchoring element 1. The coupling device includes a receiving part 5 for receiving the rod 100 and the head 3 of the bone anchoring element and a pressure element 6 for exerting pressure onto the inserted head 3 of the bone anchoring element to clamp and finally lock the head 3 in the receiving part 5. Additionally, a clamping element 7 is provided that serves for exerting a compression force onto the pressure element 6 to increase the pressure force onto an inserted head 3. The bone anchoring device further includes a locking element 8 for securing the rod 100 in the receiving part 5 and for exerting a force to lock the head 3 in the receiving part 5. The locking element may be, for example, a set screw.

The receiving part 5 is now explained with reference to Figs. 1 to 6. The receiving part 5 comprises a first end 5a and a second end 5b opposite the first end and an axis of symmetry C passing through the first end 5a and the second end 5b. A passage 51 is provided that extends from the first end 5a to the second end 5b and that is substantially rotationally symmetric to the axis of symmetry C. In a first region adjacent to the first end 5a, the receiving part 5 comprises a substantially U-shaped recess 52 that is symmetric with respect to the axis C, the recess 52 having a bottom directed towards the second end 5b. By the recess 52, two free lateral legs 53a, 53b are formed that extend towards the first end 5a. In the region of the legs 53a, 53b an internal thread 54 is provided that cooperates with the locking element 8. The internal thread 54 may be a flat thread as shown to eliminate spreading of the legs 35a, 53b when tightening the locking element 8. The internal thread may, however, have any other suitable thread form. At the end of the internal thread 54 in the direction to the second end 5b an undercut 54a may be provided with a depth in radial direction that is substantially the same as the depth of the thread 54. A channel formed by the substantially U-shaped recess 52 is sized so as to receive the rod 100 therein, wherein the rod is configured to connect a plurality of anchoring devices.

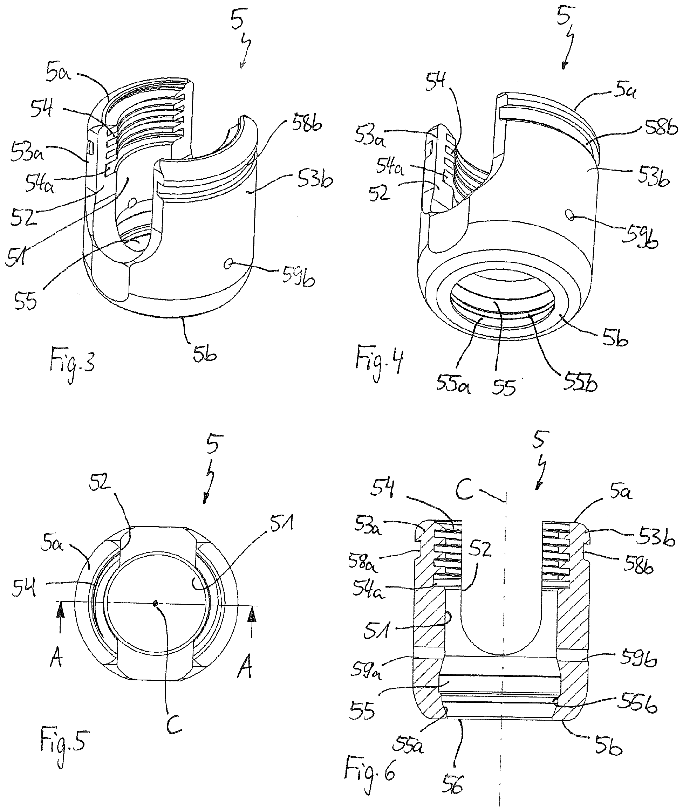

As can be seen in particular in Figs. 2 and 6, the passage 51 is between the top end 5a and approximately the bottom of the U-shaped recess 52 substantially cylindrical. From approximately the bottom of the U-shaped recess 52, the passage widens, for example conically, to form an accommodation space 55 that has an inner diameter greater than an inner diameter of the passage 51 in the upper section of the receiving part 5. The accommodation space 55 narrows towards the second end 5b with a narrowing portion 55a extending from a projecting edge 55b to the second end 5b. The size of the accommodation space is such that the head 3 of the bone anchoring element 1 and a lower portion of the pressure element 6 can be accommodated therein. The passage 51 forms an opening 56 at the bottom end 5b that opens into the accommodation space 55. A diameter of the opening 56 is greater than a greatest diameter of the head 3 so that the head 3 is insertable through the opening 56 at the bottom end 5b into the receiving part 5. It should be noted that the narrowing portion 55a can narrow in several shapes, such as in a tapered shape as shown in the Figures, in a spherical shape or can narrow otherwise.

At a distance from the first end 5a a circumferentially extending notch 58a, 58b with downwardly inclined upper and lower surfaces may be provided for engagement with a tool.

On each leg 53a, 53b bores 59a, 59b extending through the legs 53a, 53b, respectively, are provided for receiving pins 9a, 9b. The bores 59a, 59b are located approximately at a center of each leg in circumferential direction and are offset from each other by substantially 180°.

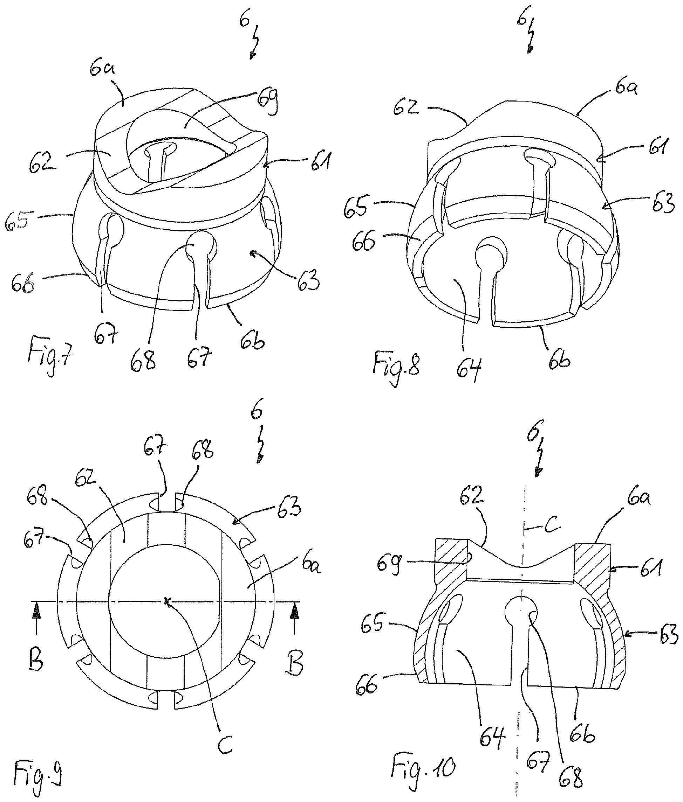

Referring further to Figs. 7 to 10, the pressure element 6 has a first end 6a and an opposite second end 6b, wherein the pressure element 6 is configured to be mounted to the receiving part 5 such that the bottom end 6b is directed towards the second end 5b of the receiving part 5. Adjacent to the first end 6a the pressure element has a first portion 61 that is substantially cylindrical and that has an outer diameter that is smaller than an inner diameter of the passage 51. In the cylindrical first portion 61 a recess with a substantially V-shaped cross-section is formed that provides a support surface 62 for supporting an inserted rod 100. The V-shape permits to support rods of different diameters such that any rod to be supported contacts the surface 62 at least two contact lines extending perpendicular to the cylinder axis of the first portion 61 which coincides in the mounted state with the central axis of symmetry C.

Between the cylindrical first portion 61 and the second end 6b there is a second portion 63 with hollow interior 64 having a shape that is adapted to clamp the head 3 therein. In particular, the hollow interior 64 has a spherical shape with such a length in axial direction that it can accommodate the head 3 with its greatest diameter e (see Fig. 2) therein. The second portion 63 is open at the second end 6b to allow the insertion of the head 3 of the bone anchoring element from the second end 6b. An outer wall of the second portion 63 comprises a first outer surface portion 65 that is substantially spherical and a second outer surface portion 66 adjacent to the second end 6b that is narrowing towards the second end 6b, in particular, in the embodiment shown it is tapered. As can be seen in Fig. 2, an outer diameter of the pressure element 6 in the region of a largest outer diameter of the first outer surface portion 65 is smaller than a greatest inner diameter of the accommodation space and greater than an inner diameter of the opening 56 of the receiving part 5. The second portion 63 of the pressure element 6 further comprises at least one vertical slit 67, preferably a plurality of slits 67 that are open to the second end 6b and extend through the second portion 63 almost up to the first portion 61. The at least one vertical slit 67 may widen into a substantially circular portion 68 forming a closed end of the slit. The number and dimensions of the slits 67 are such that the wall of the second portion 63 is flexible enough to snap onto the head 3 when the head 3 is being inserted. The enlarged end portion of the slit 68 can have another shape, for example an oval shape or any other shape that is different from the slit 67. It can even be omitted, so that the slit 67 has the same width at its closed end.

Further, the pressure element 6 comprises a coaxial bore 69 for providing access to the screw head 3 by a tool (not shown) that is configured to engage the engagement recess 3a. The second portion 63 of the pressure element 6 is a flexible portion that is adapted to exert a pressure onto an inserted head 3 and to hold the head 3 by a frictional force between the inner surface of the hollow interior 64 and the outer surface of the head 3. In other words, the flexible portion 63 is a cap-like portion that fits tightly onto the head 3. It shall be noted that the second outer surface portion 66 may have another shape, it can be rounded or have any other shape that is suitable to cooperate with the narrowing portion 55a of the receiving part 5 such that the lowermost portion of the pressure element 6 can be compressed when it enters into the narrowing portion 55a of the receiving part 5.

The pressure element 6 is configured to be inserted into the receiving part 5 through the lower opening 56, whereby the second portion 63 is compressed during insertion. Alternatively, the pressure element may be inserted through the first end 5a and the passage 51. The outer diameter of the cylindrical first portion 61 of the pressure element 6 is smaller than the inner diameter of the passage 51 to such an extent that the clamping element 7 fits there-between. Because the second portion 63 of the pressure element 6 has an outer diameter that is smaller than an inner diameter of the accommodation space 55 and because the second portion 63 has a flexible wall, the second portion 63 can expand within the accommodation space 55 when the screw head 3 is inserted.

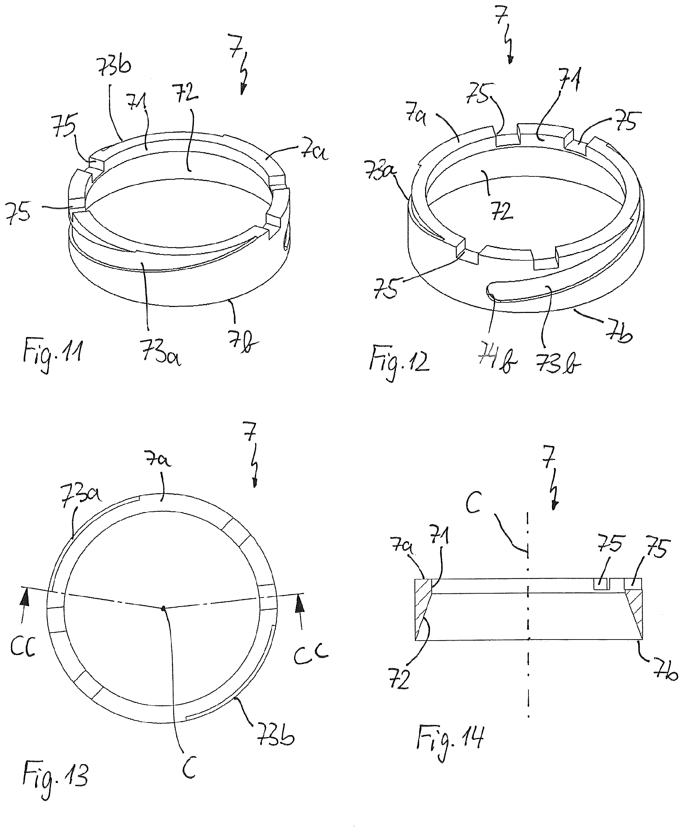

Referring further to Figs. 11 to 14, the clamping element 7 is a ring-shaped part. The ring is closed and is not flexible. The clamping element 7 has a first end 7a and an opposite second end 7b and a substantially cylindrical outer shape. As depicted in Fig. 2, the clamping element 7 is configured to be mounted to the receiving part 5 with the second end 7b showing in the direction of the second end 5b of the receiving part. The cylinder axis of the clamping element 7 coincides in the mounted state with the central axis of symmetry C of the receiving part 5. As depicted in Fig. 2, an outer diameter of the clamping element 7 is slightly smaller than an inner diameter of the passage 51 in the upper section of the receiving part 5, such that the clamping element 7 can be inserted from the first end 5a through the passage 51.

As can be seen best in Figs. 2 and 14, adjacent to the first end 7a an inner surface 71 of the clamping element is cylindrical with an inner diameter being slightly larger than an outer diameter of the first portion 61 of the pressure element 6 such that the cylindrical surface 71 of the ring-shaped clamping element 7 encompasses the outer cylindrical surface of the first portion 61 of the pressure element 6 in the mounted state. Between the cylindrical inner surface 71 and the second end 7b the clamping element comprises a conically widening portion 72. The size of the conically widening portion 72 is such that when the clamping element 7 is mounted, the portion 72 encompasses an upper region of the flexible second portion 63 of the pressure element 6. Thereby the conical surface 72 contacts the outer spherical surface 65 of the second flexible portion 63 of the pressure element 6. By a downward movement of the clamping element 7 the flexible second portion 63 of the pressure element 6 is compressed. The portion 72 may have another shape that reflects an increase in diameter towards the second end 7b.

Referring in particular to Figs. 11 and 12, two helical grooves 73a, 73b having the same pitch are provided on the outer surface of the clamping element 7. The helical grooves 73a, 73b are open towards the first end 7a and have a closed end 74a, 74b towards the second end 7b. The closed end 74a, 74b may have a rounded contour. A width of the grooves 73a, 73b is only slightly larger than the diameter of the pins 9a, 9b. In particular, the width of the grooves 73a, 73b in relation to the diameter of the pins 9a, 9b and the pitch may be preferably such that the frictional force between the pin 9a, 9b and the grooves 73a, 73b results in self-locking which means that the clamping element 7 can advance relative to the pins only if a force is applied that exceeds the self-locking force. An inclination of the groove 73 and a length of the groove between the open end and the closed end 74 is such that a desired advancement of the clamping element 7 in relation to an inserted pin 9a, 9b is achieved. In the example depicted in Figs. 11 and 12, the each groove extends less than around 180° but more than around 90° around the central axis C. However, any other inclination and length may be selected such that the clamping element 7 can be moved relative to the inserted pins 9a, 9b with a predetermined increment. The grooves 73a, 73b and the pins 9a, 9b form an advancement structure that permits to advance the clamping element 7 in the receiving part 5 through rotating the clamping element 7 around the axis of symmetry C. When the pressure element 6 and the clamping element 7 are inserted into the receiving part 5, the closed ends 74a, 74b of the helical grooves 73a, 73b prevent the clamping element 7 to escape through the top end 5a. Hence, the closed ends 74a, 74b form a securing structure.

As further depicted in Figs. 11 to 14, the clamping element 7 comprises in the surface of the first end 7a a plurality of engagement recesses 75 that are configured to be engaged with a tool used for rotating of the clamping element 7.

The parts of the bone anchoring device can be made of a bio-compatible material, such as a bio-compatible metal or a bio-compatible metal alloy, for example stainless steel, titanium, NiTi-alloys, such as Nitinol, magnesium or magnesium alloys or from a bio-compatible plastic material, such as, for example, polyether ether ketone (PEEK) or poly-1-lactide acid (PLLA). The parts can be made of the same or of different materials.

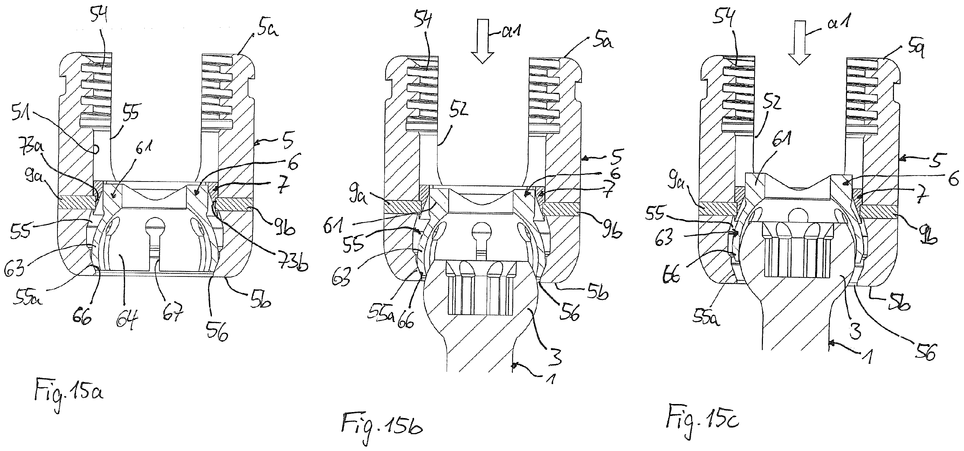

Referring to Figs. 15a to 15c, steps of assembling the bone anchoring device are explained. In Fig. 15a the pressure element 6 and the clamping element 7 are inserted into the receiving part 5. The pressure element 6 is in a position, in which the second outer surface portion 66 of the pressure element abuts against the inner surface portion 55a of the receiving part 5. The pressure element 6 may be inserted from the lower opening 56 or through the first end 5a and the passage 51. The clamping element 7 is mounted such that the pins 9a, 9b rest in the closed end portion 74a, 74b of the helical groove 73a, 73b, respectively. This is the uppermost or first position of the clamping element 7. In the uppermost position, the clamping element 7 is arranged around the cylindrical first portion 61 of the pressure element and does not contact the flexible second portion 63. Next, as depicted in Fig. 15b, the head 3 of the bone anchoring element is inserted into the pressure element 6 and the receiving part 5 through the lower opening 56 of the receiving part. In a first alternative, the bone anchoring device is assembled in-situ. This means, that the bone anchoring element 1 has been already inserted into the bone and the coupling device including the receiving part 5 with the pressure element 6 and the clamping element 7 is mounted onto the head 3 (arrow a1). In a second alternative, the bone anchoring device is assembled outside the human body and the head 3 is inserted manually into the receiving part through the lower opening 56.

Next, as depicted in Fig. 15c, the head 3 moves the pressure element 6 upward and snaps into the hollow interior 64 of the flexible portion 63 of the pressure element 6. When the head 3 with the greatest diameter e passes the second end 6b of the pressure element and moves into the hollow interior 64, the flexible portion 63 can expand in the space provided in the accommodation space. During this step, the clamping element 7 stays in its position and cannot be pushed upward as it is held by the pins 9a, 9b.

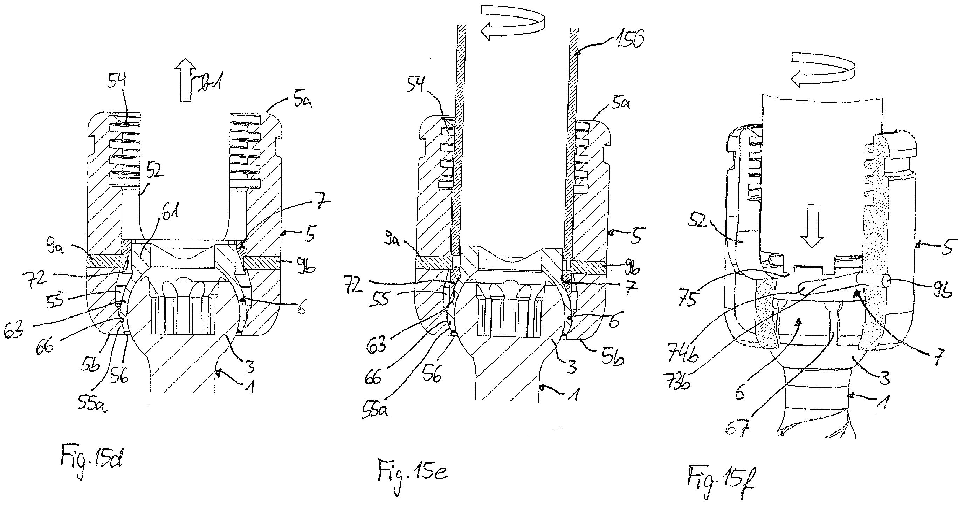

Figs. 15d to 15f illustrate steps of achieving and adjusting the frictional clamping of the head 3. As depicted in Fig. 15d, when the head 3 has been fully inserted into the pressure element, the receiving part 5 is pulled upwards (arrow b1), whereby the pressure element together with the inserted head 3 is moved downward. The lowermost portion 66 of the pressure element 6 passes the edge 55b protruding into the accommodation space 55 and enters the narrowing portion 55a whereby it is compressed and the head 3 is clamped by friction. In addition, the head 3 can no longer be pulled through the lower opening due to the pressure element 6 narrowing the lower opening 56. This position is a pre-locking position of the pressure element 6. In order to allow in-situ mounting of the receiving part 5 the insertion force for inserting the head 3 into the receiving part 5 should not be too high. The achieved friction of the head 3 with respect to the pressure element 6 after insertion of the head might not be strong enough for convenient handling.

To enhance the frictional clamping of the head 3 the clamping element 7 is actuated in a next step using a tool 150 as shown in Figs. 15e and 15f. The tool 150 may be formed as a tubular rod with engagement portions at the front side that are arranged and sized so as to fit in the engagement portions 75 of the clamping element 7. The clamping element 7 is rotated by rotating the tool 150 so that the pins 9a, 9b are guided in the helical grooves 73a, 73b. Thereby, the clamping element 7 advances downward until its inner surface portion 72 contacts the outer first surface portion 65 of the pressure element 6. Further rotating of the tool 150 advances the clamping element 7 such that it exerts a compression force onto the flexible second portion 63 of the pressure element 6 which increases the pressure of the pressure element 6 onto the inserted head 3. As a result thereof, the frictional force that holds the head 3 in a desired angular position before final locking is increased. Due to the bayonet-like advancement structure a stepless advancement of the clamping element 7 is permitted which in turn permits a stepless adjustment of the compression force onto the head 3. Fig. 15f depicts a partial cross-sectional view which illustrates how the associated groove 73b has moved relative to the mounted pin 9b. With a self-locking design of the groove relative to the pin a desired position of the clamping element can be selected and maintained.

Finally, after alignment of the receiving part of several bone anchoring devices the rod 100 is inserted and the locking element 8 is tightened. Thereby, the pressure element 6 is pressed further against the narrowing portion 55a which locks the whole construct.

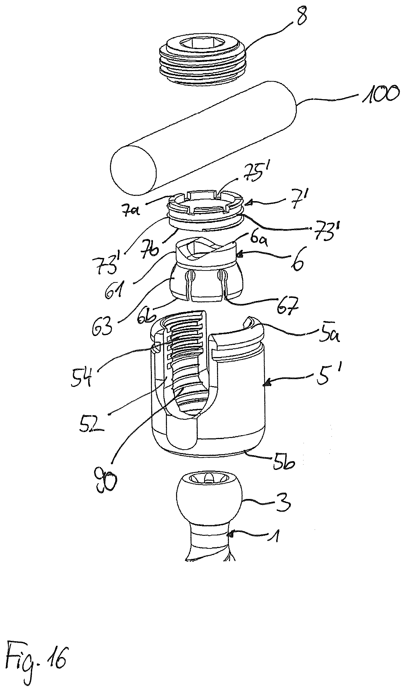

A second embodiment of the bone anchoring device and of the coupling device will be described with reference to Figs. 16 to 23. The description of parts, portions and elements that are identical or highly similar to those of the first embodiment will not be repeated. The second embodiment differs from the first embodiment in the design of the clamping element and the advancement structure. All other parts are the same as in the first embodiment.

As depicted in Figs. 16 to 20, the receiving part 5' comprises an internal thread 90 that extends from the end of the internal thread 54 in the direction of the accommodation space 55 to an axial height of approximately slightly below the bottom of the U-shaped recess 52. The internal thread 90 may be separated from the internal thread 54 by the undercut portion 54a. A thread pitch of the internal thread 90 is substantially the same as a thread pitch of the internal thread 54 and an outer diameter of the internal thread 90 is smaller than an inner diameter of the internal thread 54. The shape of the thread may be different from the shape of the internal thread 54. For example, the thread flanks may have a triangular cross-section or any other cross-section. Howver, the dimensions of the threads should be such that the clamping element can be screwed through the upper portion of the passage 51 with the internal thread 54 into the portion with the thread 90. It should be noted, that in this embodiment, the receiving part 6' does not need to have pins.

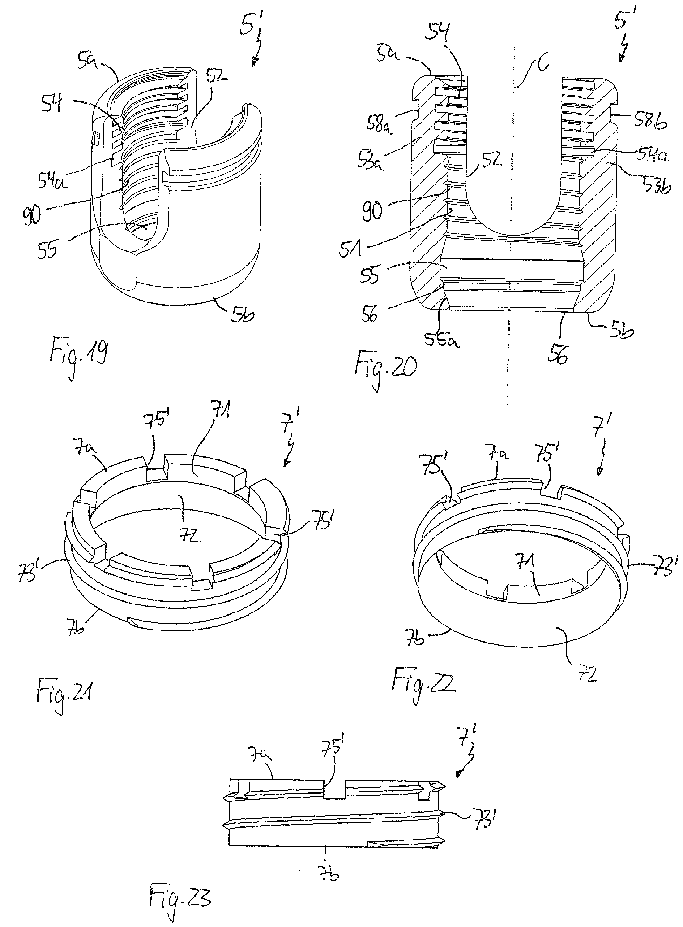

As shown in Figs. 21 to 23, the clamping element 7' comprises an external thread 73' provided on its outer cylindrical surface that is configured to cooperate with the internal thread 90 of the receiving part 5'. At the first end, the clamping element may have a plurality of engagement recesses 75'. In particular the number of engagement recesses 75' may be greater than in the first embodiment. The advancement structure in the form of the cooperating threads 73', 90 also provides a step less advancement of the clamping element 7' relative to the receiving part 5.' The threaded connection between the clamping element 7' and the receiving part 5' acts as a securing structure that inhibits inadvertent movement of the clamping element 7' relative to the receiving part 5'.

In use, after the pressure element 6 has been inserted into the receiving part 5' the clamping element 7' is mounted by screwing it from the first end 5a of the receiving part 5' downward until it has an axial position that still allows to move the pressure element 6 upward to insert the head 3. Screwing-in the clamping element 7' renders the assembly simple and safe, as jamming of the clamping element in the passage 51 is prevented thereby.

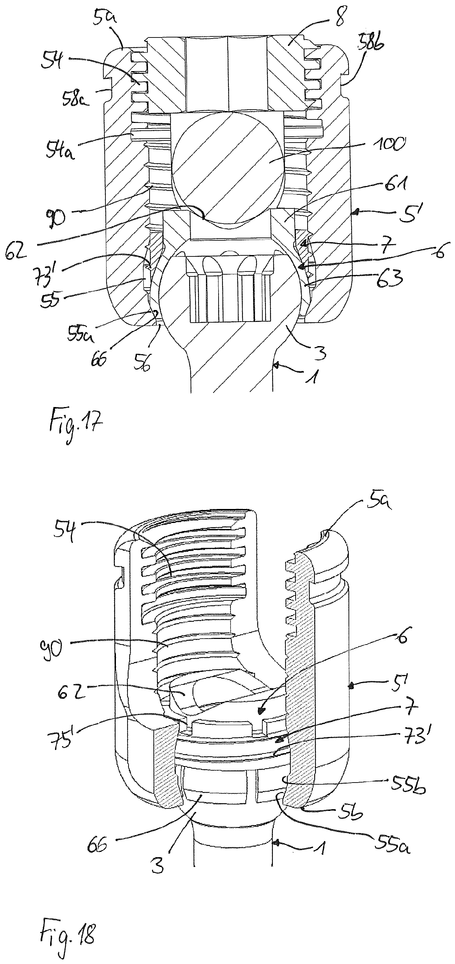

As soon as the head 3 has been inserted into the pressure element 6 and the pressure element 6 has reached the pre-locking position shown in Fig. 17, the clamping element 7 can be further screwed downward with a tool to exert a compression force onto the pressure element 6 to enhance the clamping of the head 3 by friction. After the rod 100 has been inserted and the locking element 8 has been tightened, the pressure element 6 is pressed against the narrowing portion 55a which locks the whole construct.

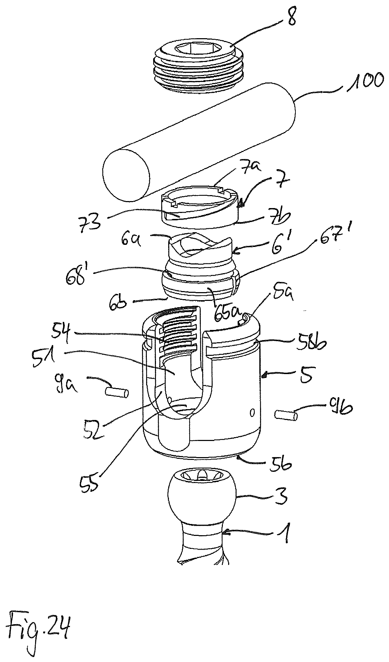

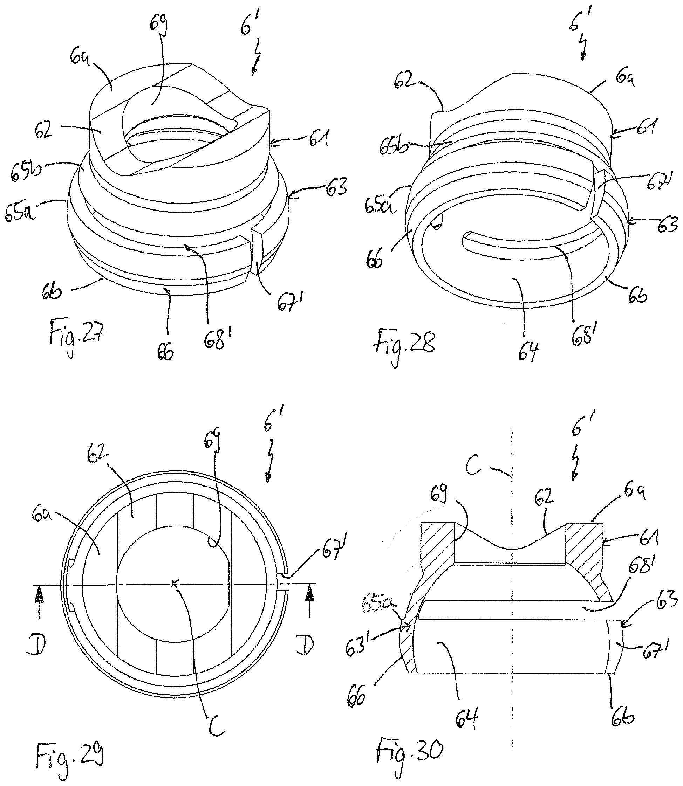

A third embodiment of the bone anchoring device will be described with reference to Figs. 24 to 30. The bone anchoring device differs from the first embodiment in the design of the coupling device and in particular in the design of the pressure element. Parts, portions and elements of the third embodiment that are identical or highly similar to those of the first embodiment, are marked with the same reference numerals and the description thereof will not be repeated. The coupling device comprises the receiving part 5 and the clamping element 7 of the first embodiment and a modified pressure element 6'. The flexible second portion 63' of the pressure element 6' comprises one single vertical slit 67' that is provided in a circumferential direction at an angle of substantially 90° relative to a longitudinal axis of the V-shaped rod support surface 62. The vertical recess 67' is open towards the second end 6b. At a distance from the second end 6b the vertical recess 67' opens into a horizontal recess 68' that extends in a circumferential direction from both sides from the vertical recess 67'. The ends of the horizontal recess 68' are spaced apart from each other so that by means of the vertical recess 67' and the horizontal recess 68' a ring 65a is formed that is connected to the remaining portion of the pressure element 6'. The ring 65a is flexible and can be expanded and/or compressed. An upper portion of the pressure element 6' that is between the horizontal recess 68' and the cylindrical portion 61 has a substantially spherical outer surface that is slightly recessed with respect to the ring 65a. In other words the ring 65a has a slightly increased outer diameter forming a circumferential edge 65b.

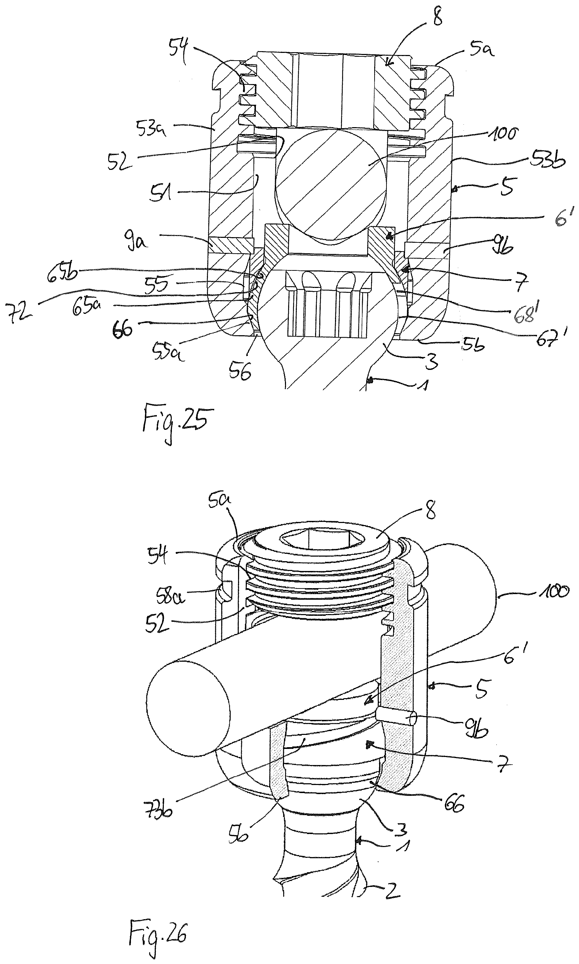

In the assembled state when the clamping element 7 has been moved downward, as depicted in Figs. 25 and 26, the clamping element 7 presses with its inner surface 72 onto the edge 65a that has the slightly greater diameter.

The use of the bone anchoring device is analogous to the use of the bone anchoring devices according to the previous embodiments. However, the horizontal slit 68' in connection with the vertical slit 67' renders the lower portion of the pressure element 6 more flexible. Therefore, an insertion force for the head 3 may be decreased. In turn, the frictional force to hold the inserted head 3 in the pressure element and the receiving part may be smaller. By means of the clamping element 7, the frictional force can be increased.

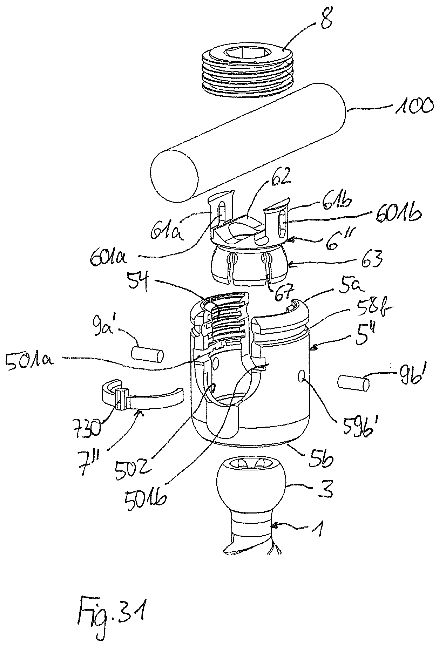

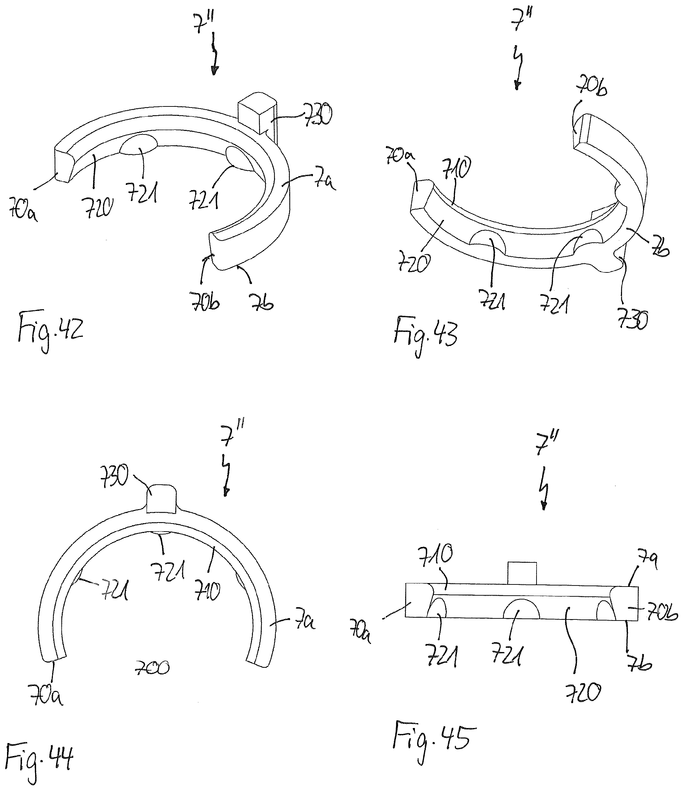

A fourth embodiment of the bone anchoring device will be explained with reference to Figs. 31 to 45. The bone anchoring device differs from the previous embodiments in the structure of the receiving part, the pressure element and the clamping element. Parts, portions and elements that are identical or highly similar to the parts, portions and elements of the previous embodiments, are indicated with the same reference numerals and the description thereof will not be repeated. Referring more in detail to Figs. 31 to 37, the receiving part 5" is adapted to accommodate the clamping element 7" according to this embodiment. The receiving part 5" includes on each leg 53a, 53b at one side from the central axis C opposite horizontal slits 501a, 501b that extend substantially perpendicular to the central axis C and are open towards the outer surface of the legs 53a, 53b. At their outer region, the recesses 501 a, 501b may have a substantially square or rectangular cross-section with a size adapted to accommodate at least a portion of the clamping element 7" therein. The recesses 501a, 501b are located at an axial position that is approximately at the lower end of the internal thread 54 which allows to mount the clamping element from the side through the U-shaped recess 52.

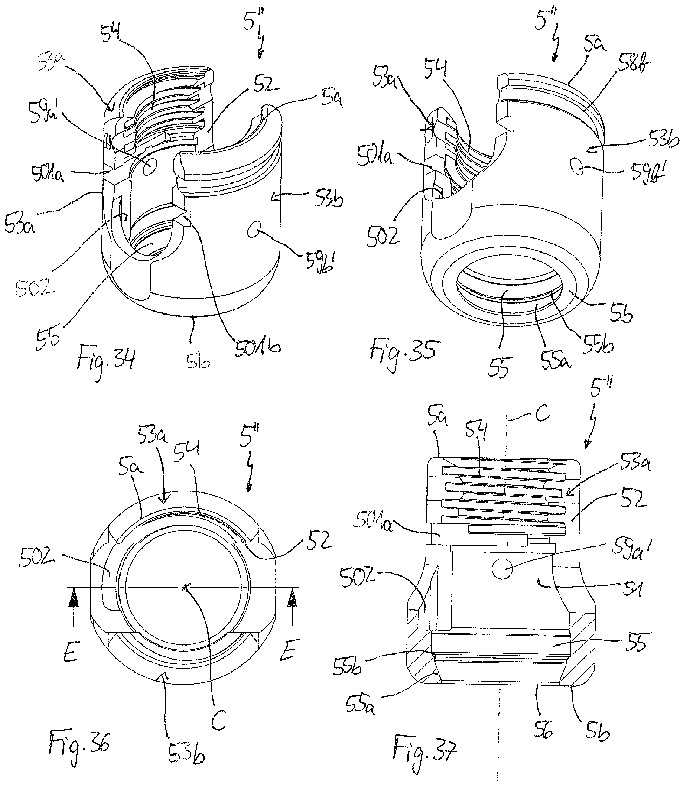

At the side of the slits 501a, 501b, a substantially vertically extending shallow recess 502 is provided that extends from the bottom of the substantially U-shaped recess 52 towards the second end 5b. In a circumferential direction the width of the shallow vertical recess 502 is slightly larger than the half of the distance between the legs 53a, 53b as can be seen in particular in Fig. 36. The shallow recess 502 serves as a space for a portion of the clamping element 7" to permit a rotating movement of the clamping element from a first a first position to a second position and to limit the movement between these positions. Moreover, two opposite bores 59a', 59b' are provided at approximately the center of the legs 53a, 53b and at an axial position slightly below the axial position of the horizontal slits 501a, 501b. The bores 59a', 59b' are configured to accommodate pins 9a', 9b', respectively, therein. The pins 9a', 9b' are configured to engage the pressure element 6" as explained below.

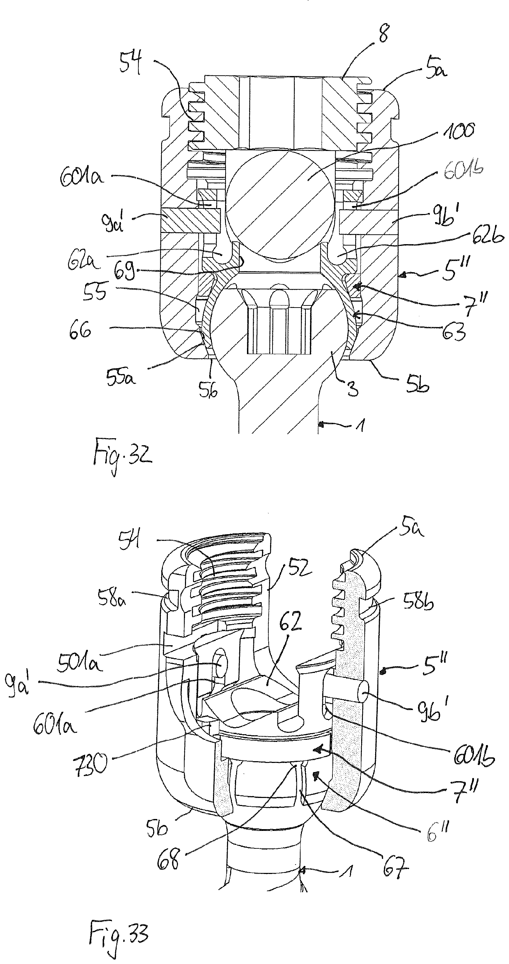

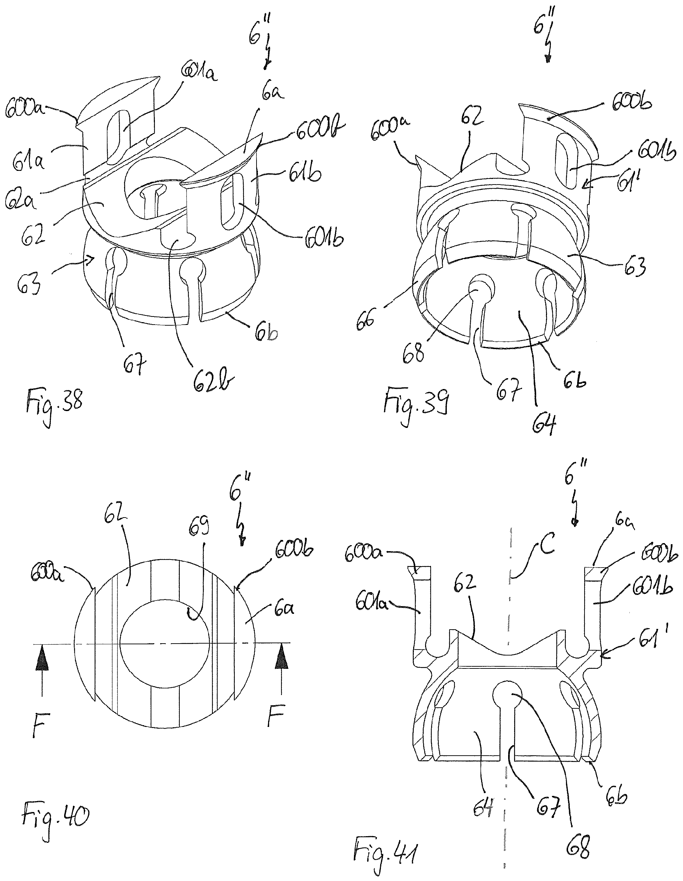

Turning now to Figs. 38 to 41, the pressure element 6" differs from the pressure element of the first and second embodiment by the shape of the upper portion adjacent to the flexible second portion. The flexible second portion 63 is identical to the flexible second portion of the first embodiment. The cylindrical first portion 61' has an outer diameter that is greater than an outer diameter of the flexible second portion in the upper region, hence, the flexible second portion 63 is recessed with respect to the first portion 61'. The first portion 61' includes two opposite upstanding legs 61 a, 61 b that are separated from an inner portion comprising the rod supporting surface 62 by a groove 62a, 62b, respectively, that may have a widened bottom to render the legs 61a, 61b slightly flexible for insertion purposes. The grooves 62a, 62b extend substantially parallel to longitudinal axis of the rod-supporting surface 62. An inner surface of the legs 61a, 61b is substantially flat. At an upper end, the legs 61a, 61b comprise each a collar portion 600a, 600b. The collar portion is shaped so as to fit in a corresponding cutout in the receiving part 5" in the region of the horizontal recesses 501a, 501b as shown in particular in Fig. 33. The legs 61 a, 61 b each comprise an elongate through-hole 601 a, 601 b the longitudinal axis of which is substantially parallel to the central axis C. The through-holes 601a, 601b are configured to receive the pins 9a', 9b', respectively. When the pins 9a', 9b' engage the through-holes 601a, 601b, the pressure element can move only a predetermined distance in axial direction limited by the abutment of the pins against the respective ends of the through-hole in axial direction.

Referring to Figs. 31 to 33 and 42 to 45, the clamping element 7" will be explained. The clamping element 7" is shaped as an open ring having free ends 70a, 70b and a slot 700 therebetween, so that the ring can act as a snap ring. More in detail, the clamping element 7" extends in circumferential direction slightly more than 180° around the central axis C. The slot 700 may be smaller or may be larger as shown. Further, the clamping element 7" has a substantially cylindrically shaped outer wall with an axial length that is such that the clamping element 7 fits approximately into the recessed region between the first portion 61' and the second portion 63' of the pressure element 6" as shown in Fig. 32. The clamping element 7" comprises a substantially conically widening inner surface portion 720 that is adapted to extend around the upper section of the flexible second portion 63' of the pressure element 6" as also depicted in Fig. 32. Between the widening section 720 and the top end 7a there may be small portion 710 that can be rounded to avoid jamming of the clamping element 7" during mounting. In the widening portion 720 there are a plurality of rounded protrusions 721 that are configured to engage the widened end portions 68 of the slits 67 of the flexible portion 63' of the pressure element 6". It shall be noted that a modification of the design of the pressure element and the clamping element may be conceivable where instead of the widened portions 68 of the slit 67 in the pressure element and the protrusions on the inner surface of the clamping element 7' protrusions could be present at the end of the slits 67 and corresponding dimples could be present in the inner surface of the clamping element.

At approximately the center in a circumferential direction there is a protrusion 730 with a substantially rectangular cross-section that is provided at the outside of the clamping element 7" opposite to the protrusions 721. In an axial direction, the protrusion 730 protrudes to some extent above the top end 7a. The protrusion 730 serves for engagement with a tool for moving the clamping element 7" relative to the pressure element 6".

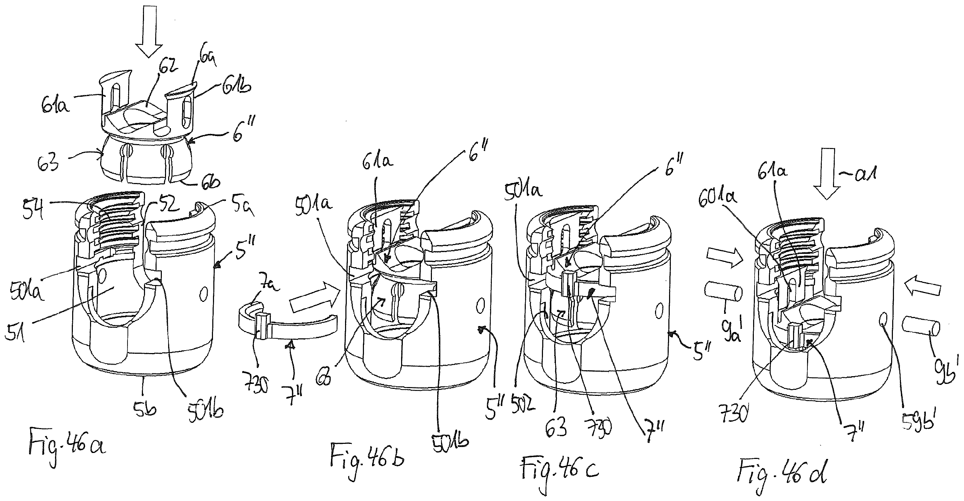

Mounting of the pressure element 6" and the clamping element 7" into the receiving part 5" will be explained with reference to Figs. 46a to 46d. In a first step, the pressure element 6" is inserted into the receiving part 5" from the first end 5a. The legs 61a, 61b may be slightly flexed towards each other such that the pressure element can be maintained at a desired axial position. When the region between the cylindrical first portion 61' and the flexible second portion 63' is located at an axial height of approximately the horizontal slits 501a, 501b, the clamping element 7" is inserted into the slits 501a, 501b of the receiving part 5" until it extends around the pressure element 6" just beneath the cylindrical first portion 61'. The protrusion 730 is aligned with the center of the substantially U-shaped recess and the rod-supporting surface 62 is also aligned with the U-shaped recess 52 of the receiving part 5". One of the slits 67 is located at a circumferential position corresponding to the center of the rod-supporting 62 of the pressure element 6". The corresponding protrusion 721 at the inner surface 720 of the clamping element 7" can engage the widened portion 68 of the slit 67 as depicted in Fig. 46c. Thereafter, as shown in Fig. 46d, the pressure element 6" and the clamping element 7" are moved downward together as indicated by the arrow a1. Thereby, the elongate through-holes 601a, 601b overlap with the bores 59a', 59b', so that the pins 9a', 9b' can be inserted into the bores and extend into the through-holes 601a, 601b.

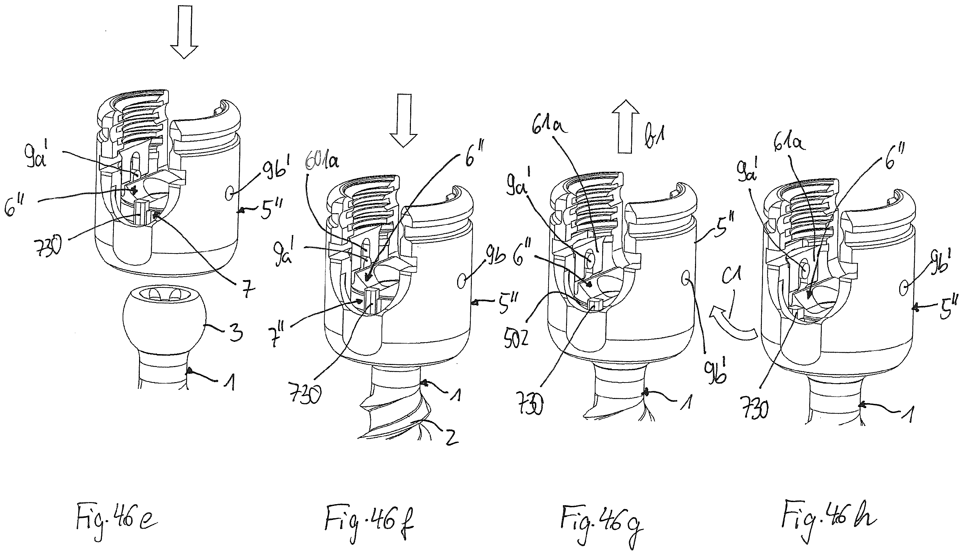

Use of the bone anchoring device will be explained referring to Figs. 46e to 46h. First, as depicted in Fig. 46e, the receiving part 5" with the mounted pressure element 6" and clamping element 7" is placed onto the head 3 of the bone anchoring element 1. This may be performed in-situ while the bone anchoring element has been already inserted into the bone. As described in the previous embodiments, the pressure element 6" snaps onto the head 3 so that the head 3 is accommodated in the pressure element and in the accommodation space 55 of the receiving part as shown in Fig. 46f. Thereafter, as illustrated in Fig. 46g, the receiving part is pulled upward according to the arrow b 1. The pins 9a', 9b' move upward in the elongate through-holes 601 a, 601b, respectively. The protrusion 730 of the clamping element 7" disappears partially in the shallow recess 502. In this manner, the pre-locking position has been achieved in which it is no longer possible to remove the head 3 through the lower opening 56.

Thereafter, as shown in Fig. 46h, the clamping element 7" is rotated in the clockwise direction around the central axis C (arrow c1). The rotation can be effected using a tool (not shown) that engages the protrusion 730. Thereby, the rounded protrusions 721 move out of the recesses 68 of the pressure element and press against the flexible second surface portion 63' of the pressure element 6". By means of this compression, the clamping force onto the head 3 is increased. The rotational movement is limited by the abutment of the protrusions 730 against the sidewall of the shallow recess 502. The pins 9a', 9b' prevent the pressure element 6" from escaping out of the receiving part 5".

The steps of insertion of the rod 100 and of the locking element 8 and tightening the locking element 8 to lock the whole construct are identical to the previous embodiments.

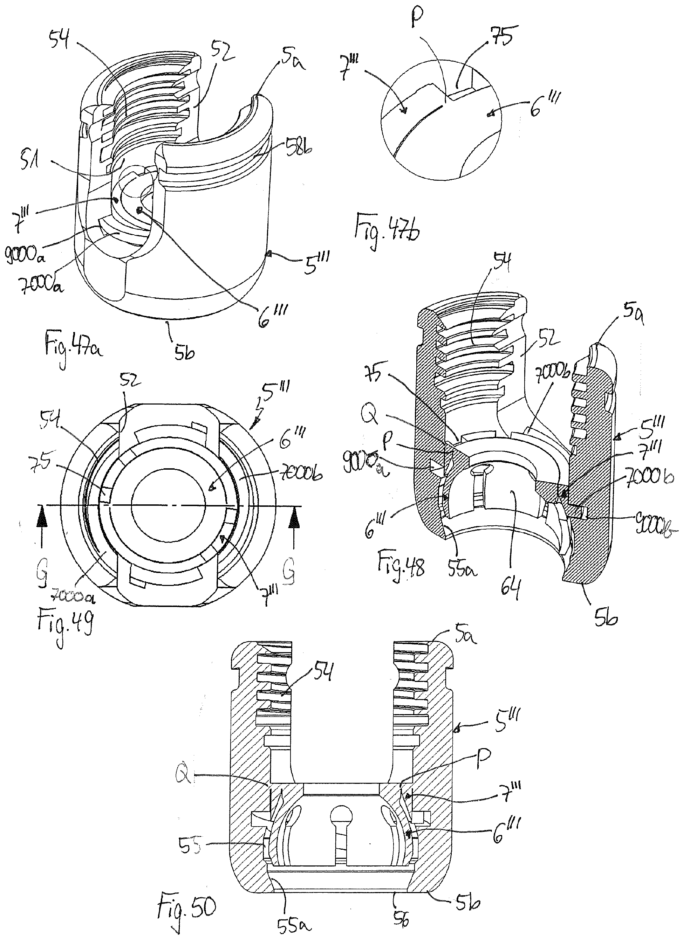

A fifth embodiment of the polyaxial bone anchoring device and the coupling device will be described with reference to Figs. 47a to 50. The bone anchoring element is identical to the previous embodiments. The receiving part 5"', the clamping element 7"' and the pressure element 6"' are manufactured as a monolithic piece and separated after manufacturing. The pressure element has a shape like the pressure element in the first embodiment. The clamping element is similar to the clamping element of the first and second embodiment, however, instead of the two grooves 73a, 73b the clamping element 7"' comprises on its outer surface two helical projections 7000a, 7000b. These helical projections are configured to engage a corresponding helical grooves 9000a, 9000b in the receiving part 5"'. As shown in more detail in Figs. 47a and 47b, the clamping element 7"' is monolithically connected to the pressure element 6"' at predetermined breaking points P. The predetermined breaking points have a size such that by engagement of the clamping element 7"' with a tool that cooperates with the recesses 75 and rotating the clamping element, the predetermined breaking points P break so that the clamping element 7"' and the pressure element 6"' become separated. Similarly, the clamping element 7"' is connected to the receiving part 5"' monolithically at predetermined breaking points Q as shown in Figs. 48 and 50, wherein the size of the breaking points Q is such that by rotation of the clamping element with a tool the connection between the receiving part 5"' and the clamping element 7"' breaks at the predetermined breaking points Q.

The receiving part 5"', the pressure element 6"' and the clamping element 7" may be separated before mounting the coupling device to a bone anchoring element. In particular, the pressure element 6"' and the clamping element 7"' may be manufactured such that the position of the pressure element is the inserting position for inserting the head 3. The separation can take place during mounting of the receiving part 5"' onto the head 3 of the bone anchoring element 1 in-situ.

A method for manufacturing the coupling device may be an additive manufacturing method such as selective laser sintering, selective laser melting, electron beam-sintering and electron beam-melting.

Modifications of the above described embodiments may be possible. For the bone anchoring element all kinds of bone anchors can be used, such as screws, nails with or without barbs, cannulated bone anchors, two-part bone anchors, where head and shank are separate parts that can be assembled and other bone anchoring elements. The head of the bone anchoring element may have a design that allows the bone anchoring element with a pressure element adapted thereto to be pivoted only in a single plane. For example, the head may have at least one flat surface portion extending substantially parallel to the shank axis and the pressure element may have a cooperating portion to limit the pivoting to a single plane. Any design for providing an enlarged pivot angle may be used also.

For the locking element all kinds of locking devices can be used, such as bayonet-type locking devices, two-part locking devices that allow to clamp the rod and the head independently with two locking elements, outer locking nuts, etc..

While some of the embodiments include two pins it shall be understood that one pin is sufficient.

The features of the embodiments describe can be combined among each other to provide a variety of still further embodiments.

## Claims

1. A coupling device for coupling a rod to a bone anchoring element, the coupling device including
a receiving part (5, 5', 5", 5"') having a first end (5a) and a second end (5b), a central axis (C) extending through the first end and the second end, a channel (52) for receiving a rod, wherein the receiving part defines an accommodation space (55) for accommodating a head (3) of a bone anchoring element (1), the accommodation space (55) having an opening (56) for inserting the head; and
a pressure element (6, 6', 6", 6"') arranged at least partially in the accommodation space (55), the pressure element comprising a flexible portion (63, 63') to clamp an inserted head,
a clamping element (7, 7', 7", 7"') extending at least partially around the flexible portion (63, 63'), wherein the clamping element (7, 7', 7", 7"') is configured to move from a first position to a second position in which it exerts a clamping force onto the pressure element (6, 6', 6", 6"'),
**characterized in that** the movement includes rotating the clamping element (7, 7', 7", 7"') around the central axis (C).

2. The coupling device of claim 1, wherein when the clamping element (7, 7', 7", 7"') is in the first position the clamping force exerted onto the pressure element (6, 6', 6", 6"') is smaller than the clamping force exerted in the second position or no clamping force is exerted onto the pressure element.

3. The coupling device of claim 1 or 2, wherein the pressure element (6, 6', 6", 6"') has a cap-like shape and is configured to cover an inserted head (3) from a side opposite to the shank (2).

4. The coupling device of claim 3, wherein the pressure element (6, 6', 6", 6"') is formed such that it extends beyond a largest diameter (e) of an inserted head (3) towards the second end (5b) of the receiving part (5, 5', 5", 5"') and wherein the clamping element (7, 7', 7", 7"') is provided at an axial position above the largest diameter in the direction of the first end (5a).

5. The coupling device of one of claims 1 to 4, wherein the clamping element (7, 7', 7", 7"') comprises an engagement portion (75, 75', 730) for engagement with a tool to move from the first position to the second position to adjust the clamping force.

6. The coupling device of one of claims 1 to 5, wherein an advancement structure (73a, 73b, 9a, 9b, 73', 90, 7000a, 7000b, 9000a, 9000b) is provided that is configured to permit an axial advancement of the clamping element (7, 7', 7"') when the clamping element is rotated about the central axis (C).

7. The coupling device of claim 6, wherein the advancement structure comprises a helical groove (73a, 73b; 73', 9000a, 9000b) in an outer wall of the clamping element (7, 7', 7"') or an inner wall of the receiving part (5"') and an engagement member (9a, 9b, 90, 7000a, 7000b) at an inner wall of the receiving part or at an outer wall of the clamping element for engaging the helical groove.

8. The coupling device of claim 7, wherein the engagement member is at least one pin (9a, 9b) configured to engage the groove (73a, 73b).

9. The coupling member of claim 6 or 7, wherein the advancement structure comprises a thread (73') on an outer surface of the clamping element cooperating with a thread (90) at an inner wall of the receiving part (5').

10. The coupling device of one of claims 1 to 9, wherein the pressure element (6, 6', 6", 6"') and the clamping element (7, 7', 7", 7"') are separate parts.

11. The coupling device of one of claims 1 to 10, wherein the pressure element (6"') and the clamping element (7"') and preferably also the receiving part (5"') are manufactured as a monolithic piece, for example through additive manufacturing, and are configured to be separated from each other at predetermined breaking points (P, Q).

12. The coupling device of one of claims 1 to 11, wherein the clamping element is shaped as a slotted ring (7") that extends partially around the pressure element.

13. The coupling device of claim 12, wherein the clamping element (7") comprises protrusions (721) or recesses at its side facing the pressure element (6") that may cooperate with recesses (68) or protrusions at the pressure element (6") to secure the first position of the clamping element (7").

14. The coupling device of one of claims 1 to 13, wherein the pressure element (6, 6', 6", 6"') comprises at least one axial slot (67, 67') to provide flexibility to the flexible portion (63, 63') and wherein preferably a horizontal slot (68') may be provided.

15. A bone anchoring device comprising a coupling device of one of claims 1 to 14 and a bone anchoring element (1) with a shank (2) and a head (3) wherein the head preferably has a spherical outer surface portion.

## Patentansprüche

1. Kopplungsvorrichtung zum Koppeln eines Stabs an ein Knochenverankerungselement, wobei die Kopplungsvorrichtung umfasst
ein Aufnahmeteil (5, 5', 5", 5"'), welches ein erstes Ende (5a) und ein zweites Ende (5b), eine Zentralächse (C), die sich durch das erste Ende und das zweite Ende erstreckt, und einen Kanal (52) zum Aufnehmen eines Stabs aufweist, wobei das Aufnahmeteil einen Aufnahmeraum (55) zum Aufnehmen eines Kopfs (3) eines Knochenverankerungselements (1) festlegt, wobei der Aufnahmeraum (55) eine Öffnung (56) zum Einführen des Kopfs aufweist; und
ein Druckelement (6, 6', 6", 6"'), welches zumindest teilweise in dem Aufnahmeraum (55) angeordnet ist, wobei das Druckelement einen flexiblen Abschnitt (63, 63') zum Klemmen eines eingeführten Kopfs aufweist,
ein Klemmelement (7, 7', 7", 7"'), welches sich zumindest teilweise um den flexiblen Abschnitt (63, 63') herum erstreckt, wobei das Klemmelement (7, 7', 7", 7"') ausgebildet ist, um sich von einer ersten Position zu einer zweiten Position, in welcher es eine Klemmkraft auf das Druckelement (6, 6', 6", 6"') ausübt, zu bewegen,
**dadurch gekennzeichnet, dass** die Bewegung eine Drehung des Klemmelements (7, 7', 7", 7"') um die Zentralachse (C) herum einschließt.

2. Kopplungsvorrichtung gemäß Anspruch 1, wobei, wenn sich das Klemmelement (7, 7', 7", 7"') in der ersten Position befindet, die Klemmkraft, welche auf das Druckelement (6, 6', 6", 6"') ausgeübt wird, kleiner ist als die Klemmkraft, welche in der zweiten Position ausgeübt wird, oder keine Klemmkraft auf das Druckelement ausgeübt wird.

3. Kopplungsvorrichtung gemäß Anspruch 1 oder 2, wobei das Druckelement (6, 6', 6", 6"') eine kappenähnliche Form aufweist und ausgebildet ist, um einen eingeführten Kopf (3) von einer dem Schaft (2) gegenüberliegenden Seite zu bedecken.

4. Kopplungsvorrichtung gemäß Anspruch 3, wobei das Druckelement (6, 6', 6", 6"') so geformt ist, dass es sich über einen größten Durchmesser (e) eines eingeführten Kopfs (3) in Richtung des zweiten Endes (5b) des Aufnahmeteils (5, 5', 5", 5"') hin erstreckt und wobei das Klemmelement (7, 7', 7", 7"') an einer axialen Position oberhalb des größten Durchmesser in Richtung des ersten Endes (5a) hin vorgesehen ist.

5. Kopplungsvorrichtung gemäß einem der Ansprüche 1 bis 4, wobei das Klemmelement (7, 7', 7", 7"') einen Eingreifabschnitt (75, 75', 730) zum Eingreifen mit einem Instrument umfasst, um sich von einer ersten Position zu einer zweiten Position zu bewegen, um die Klemmkraft einzustellen.

6. Kopplungsvorrichtung gemäß einem der Ansprüche 1 bis 5, wobei eine Vortriebstruktur (73a, 73b, 9a, 9b, 73', 90, 7000a, 7000b, 9000a, 9000b) vorgesehen ist, welche ausgebildet ist, um ein axiales Vortreiben des Klemmelements (7, 7', 7") zu erlauben, wenn das Klemmelement um die Zentralachse (C) herum rotiert wird.

7. Kopplungsvorrichtung gemäß Anspruch 6, wobei die Vortriebstruktur eine helikale Nut (73a, 73b; 73', 9000a, 9000b) in einer äußeren Wandung des Klemmelements (7, 7', 7"') oder in einer inneren Wandung des Aufnahmeteils (5"') und ein Eingreifelement (9a, 9b, 90, 7000a, 7000b) an einer inneren Wandung des Aufnahmeteils oder an einer äußeren Wandung des Klemmelements zum Eingreifen in die helikale Nut umfasst.

8. Kopplungsvorrichtung gemäß Anspruch 7, wobei das Eingreifelement mindestens ein Stift (9a, 9b) ist, welcher ausgebildet ist, um in die Nut (73a, 73b) einzugreifen.

9. Kopplungsvorrichtung gemäß Anspruch 6 oder 7, wobei die Vortriebstruktur ein Gewinde (73') auf einer äußeren Fläche des Klemmelements umfasst, welches mit einem Gewinde (90) an einer inneren Wandung des Aufnahmeteils (5') zusammenwirkt.

10. Kopplungsvorrichtung gemäß einem der Ansprüche 1 bis 9, wobei das Druckelement (6, 6', 6", 6"') und das Klemmelement (7, 7', 7", 7"') getrennte Teile sind.

11. Kopplungsvorrichtung gemäß einem der Ansprüche 1 bis 10, wobei das Druckelement (6"') und das Klemmelement (7"') und vorzugweise auch das Aufnahmeteils (5"') einstückig hergestellt sind, beispielsweise durch ein generatives Fertigungsverfahren, und ausgebildet sind, an Sollbruchstellen (P, Q) voneinander getrennt zu werden.

12. Kopplungsvorrichtung gemäß einem der Ansprüche 1 bis 11, wobei das Klemmelement als ein geschlitzter Ring (7") geformt ist, welcher sich teilweise um das Druckelement herum erstreckt.

13. Kopplungsvorrichtung gemäß Anspruch 12, wobei das Klemmelement (7") Vorsprünge (721) oder Ausnehmungen an seiner dem Druckelement (6") zugewandten Seite umfasst, welche mit Ausnehmungen (68) oder Vorsprüngen an dem Druckelement (6") zusammenwirken können, um die erste Position des Klemmelements (7") zu sichern.

14. Kopplungsvorrichtung gemäß einem der Ansprüche 1 bis 13, wobei das Druckelement (6, 6', 6", 6"') mindestens einen axialen Schlitz (67, 67') umfasst, um den flexiblen Abschnitt (63, 63') mit Flexibilität zu versehen, und wobei vorzugsweise ein horizontaler Schlitz (68') vorgesehen sein kann.

15. Knochenverankerunasvorrichtung umfassend eine Kopplungsvorrichtung gemäß einem der Ansprüche 1 bis 14 und ein Knochenverankerungselement (1) mit einem Schaft (2) und einem Kopf (3), wobei der Kopf vorzugsweise einen sphärischen Außenoberflächenabschnitt aufweist.

## Revendications

1. Dispositif d'accouplement servant à accoupler une tige à un élément d'ancrage osseux, le dispositif d'accouplement comprenant
une partie de réception (5, 5', 5'', 5"') comportant une première extrémité (5a) et une seconde extrémité (5b), un axe central (C) s'étendant à travers la première extrémité et la seconde extrémité, un canal (52) destiné à recevoir une tige, la partie de réception définissant un espace formant logement (55) destiné à loger une tête (3) d'un élément d'ancrage osseux (1), l'espace formant logement (55) comportant une ouverture (56) pour l'insertion de la tête ; et
un élément de pression (6, 6', 6" 6"') disposé au moins partiellement dans l'espace formant logement (55), l'élément de pression comprenant une partie souple (63, 63') servant à fixer par serrage une tête insérée,
un élément de fixation par serrage (7, 7', 7", 7"') s'étendant au moins partiellement autour de la partie souple (63, 63'), l'élément de fixation par serrage (7, 7', 7", 7"') étant configuré pour se déplacer d'une première position à une seconde position dans laquelle il exerce une force de fixation par serrage sur l'élément de pression (6, 6', 6", 6"'),
**caractérisé en ce que** le déplacement comprend une rotation de l'élément de fixation par serrage (7, 7', 7'', 7''') autour de l'axe central (C).

2. Dispositif d'accouplement selon la revendication 1, dans lequel, lorsque l'élément de fixation par serrage (7, 7', 7", 7"') se trouve dans la première position, la force de fixation par serrage exercée sur l'élément de pression (6, 6', 6", 6"') est inférieure à la force de fixation par serrage exercée dans la seconde position ou aucune force de fixation par serrage n'est exercée sur l'élément de pression.

3. Dispositif d'accouplement selon la revendication 1 ou 2, dans lequel l'élément de pression (6, 6', 6", 6"') a une forme de coiffe et est configuré pour recouvrir une tête insérée (3) depuis un côté opposé à la queue (2).

4. Dispositif d'accouplement selon la revendication 3, dans lequel l'élément de pression (6, 6', 6", 6"') est formé de telle sorte qu'il s'étende au-delà d'un diamètre maximal (e) d'une tête insérée (3) en direction de la seconde extrémité (5b) de la partie de réception (5, 5', 5", 5'''), et dans lequel l'élément de fixation par serrage (7, 7', 7", 7"') est placé au niveau d'une position axiale au-dessus du diamètre maximal dans la direction de la première extrémité (5a).

5. Dispositif d'accouplement selon l'une des revendications 1 à 4, dans lequel l'élément de fixation par serrage (7, 7', 7", 7''') comprend une partie de mise en prise (75, 75', 730) destinée à être mise en prise avec un instrument, pour se déplacer de la première position à la seconde position afin d'ajuster la force de fixation par serrage.

6. Dispositif d'accouplement selon l'une des revendications 1 à 5, dans lequel une structure d'avancée (73a, 73b, 9a, 9b, 73', 90, 7000a, 7000b, 9000a, 9000b) est prévue, celle-ci étant configurée pour permettre une avancée axiale de l'élément de fixation par serrage (7, 7', 7"') lorsque l'élément de fixation par serrage est mis en rotation autour de l'axe central (C).

7. Dispositif d'accouplement selon la revendication 6, dans lequel la structure d'avancée comprend une rainure hélicoïdale (73a, 73b ; 73', 9000a, 9000b) dans une paroi extérieure de l'élément de fixation par serrage (7, 7', 7"') ou dans une paroi intérieure de la partie de réception (5"') et un organe de mise en prise (9a, 9b, 90, 7000a, 7000b) au niveau d'une paroi intérieure de la partie de réception ou au niveau d'une paroi extérieure de l'élément de fixation par serrage, pour une mise en prise avec la rainure hélicoïdale.

8. Dispositif d'accouplement selon la revendication 7, dans lequel l'organe de mise en prise est au moins une goupille (9a, 9b) configurée pour se mettre en prise avec la rainure (73a, 73b).

9. Organe d'accouplement selon la revendication 6 ou 7, dans lequel la structure d'avancée comprend un filetage (73') sur une surface extérieure de l'élément de fixation par serrage coopérant avec un filetage (90) au niveau d'une paroi intérieure de la partie de réception (5').

10. Dispositif d'accouplement selon l'une des revendications 1 à 9, dans lequel l'élément de pression (6, 6' , 6", 6"') et l'élément de fixation par serrage (7, 7', 7'', 7"') sont des pièces distinctes.

11. Dispositif d'accouplement selon l'une des revendications 1 à 10, dans lequel l'élément de pression (6"') et l'élément de fixation par serrage (7"') et, de préférence, également la partie de réception (5"') sont fabriqués sous forme d'une pièce monolithe, par exemple par fabrication additive, et sont configurés pour être séparés les uns des autres au niveau de points de rupture prédéterminés (P, Q).

12. Dispositif d'accouplement selon l'une des revendications 1 à 11, dans lequel l'élément de fixation par serrage est réalisé sous la forme d'une bague fendue (7") qui s'étend partiellement autour de l'élément de pression.

13. Dispositif d'accouplement selon la revendication 12, dans lequel l'élément de fixation par serrage (7") comprend des protubérances (721) ou des évidements au niveau de son côté faisant face à l'élément de pression (6") qui peuvent coopérer avec des évidements (68) ou des protubérances au niveau de l'élément de pression (6'') afin de fixer la première position de l'élément de fixation par serrage (7").

14. Dispositif d'accouplement selon l'une des revendications 1 à 13, dans lequel l'élément de pression (6, 6', 6", 6"') comprend au moins une fente axiale (67, 67') afin de conférer de la souplesse à la partie souple (63, 63'), et dans lequel, de préférence, une fente horizontale (68') peut être prévue.

15. Dispositif d'ancrage osseux comprenant un dispositif d'accouplement selon l'une des revendications 1 à 14 et un élément d'ancrage osseux (1) comportant une queue (2) et une tête (3), dans lequel la tête comporte, de préférence, une partie de surface extérieure sphérique.
